(19) Europäisches Patentamt / European Patent Office / Office européen des brevets

(11) **EP 3 613 341 A1**

(12) **EUROPEAN PATENT APPLICATION**
published in accordance with Art. 153(4) EPC

(43) Date of publication:
**26.02.2020 Bulletin 2020/09**

(21) Application number: **18788113.1**

(22) Date of filing: **18.04.2018**

(51) Int Cl.:
*A61B 5/026* (2006.01)      *A61B 5/00* (2006.01)
*A61B 5/029* (2006.01)

(86) International application number:
**PCT/JP2018/016009**

(87) International publication number:
**WO 2018/194093 (25.10.2018 Gazette 2018/43)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.04.2017 JP 2017083177**

(71) Applicants:
• **A School Corporation Kansai University
Suita-shi, Osaka 564-8680 (JP)**

• **Terumo Kabushiki Kaisha
Tokyo 151-0072 (JP)**

(72) Inventor: **SUZUKI, Satoshi
Suita-shi
Osaka 564-8680 (JP)**

(74) Representative: **Strehl Schübel-Hopf & Partner
Maximilianstrasse 54
80538 München (DE)**

(54) **BIOLOGICAL INFORMATION ESTIMATION DEVICE**

(57)   A biological information estimation device comprises: a transmission unit 12 and a transmission antenna 13 for emitting radio waves toward the heart; a receiving antenna 14 and a receiving unit 15 for receiving the radio waves which have passed through the heart, or the radio waves which have been reflected by the heart; and an estimation unit 11 for estimating the volume of blood contained in the heart, or the amount of change in the volume, based on the amplitude or phase of the radio waves received by the receiving unit 15, and the specific absorption rate of the heart.

## FIG.1

## Description

### Technical Field

This invention relates to a biological information estimation device which, under non-contact and non-restraint conditions, can estimate information on a biological tissue such as an organ or a limb.

### Background Art

So far, stroke volume and cardiac output have been measured to diagnose heart failure or confirm the effect of therapy or medication in prognosis for heart failure. Concrete examples of the method for measurement are invasive methods typified by Fick method, dye dilution test, and thermodilution using a Swan-Ganz catheter. As noninvasive methods of measurement, Kubicek's four-electrode method and diagnosis by ultrasound cardiography have been proposed. However, these methods of measurement are not used nowadays, because of the necessity for restraining a person targeted for measurement (simply, target person), or the problem of not providing a sufficient accuracy.

The inventor of the present invention, on the other hand, found that microwaves passing through the heart changed in amplitude or phase according to movements of the heart, such as contraction or dilatation (i.e., expansion). Based on this finding, the inventor proposed a heart beat detector capable of obtaining a heart beat by analyzing the microwaves passing through the heart (see Patent Document 1). The inventor also proposed an estimation device for heart volume and cardiac output which, under non-contact and non-restraint conditions, can detect changes over time in the heart volume and cardiac output of the target person (see Patent Document 2) .

In Patent Document 2, the heart was assumed to be spherical in estimating the heart volume. Thus, the device of the document was difficult to apply to organs other than the heart. For example, it was impossible for the device to measure the volume of the blood vessel and estimate the state of blood flow based on the measured volume.

Proposals have been made for technologies which measure not only cardiac output, etc., but also the amount of water accumulated in the limb by edema, the amount of water accumulated in the lung by pulmonary congestion, and the volume of the urinary bladder (simply, bladder) or the amount of change in urine in the bladder (see Patent Documents 3 to 5). Even these methods of measurement, however, pose problems such as the necessity of mounting a certain device on the target person, or the necessity of restraining the target person. Conventional technologies, as discussed above, have been unable to estimate, under non-contact and non-restraint conditions, the volume of a liquid, such as blood, contained in a tissue of the target person (living body) (the tissue such as the heart, blood vessel, lung, bladder, or limb), or the amount of change in the volume (the volume of the liquid in the tissue of the living body, and the amount of change in the volume will hereinafter be referred to as biological information).

### Prior Art Documents

#### Patent Documents

    Patent Document 1: JP-A-2013-153783
    Patent Document 2: JP-A-2016-202516
    Patent Document 3: JP-A-Hei-5-237119
    Patent Document 4: JP-T-2010-532208
    Patent Document 5: JP-A-2005-087543

### Summary of the Invention

#### Problems to be solved by the invention

The present invention has been accomplished in the light of the above-described circumstances. It is an object of the invention to provide a biological information estimation device which, under non-contact and non-restraint conditions, can estimate the volume of a liquid contained in a tissue, or the amount of change in the volume.

### Means for solving the problems

A first aspect of the present invention, intended to attain the above object, resides in a biological information estimation device comprising: radio wave transmission means for emitting radio waves toward a tissue of a living body;

radio wave receiving means for receiving the radio waves which have passed through the tissue, or the radio waves which have been reflected by the tissue; and estimation means for estimating the volume of a liquid contained in the tissue based on the amplitude or phase of the radio waves received by the radio wave receiving means, and the specific absorption rate of the tissue.

In the first aspect, the volumes of liquids contained in wide varieties of organs including the heart can be estimated based on the specific absorption rate of the tissue and the amplitude or phase of the radio waves.

A second aspect of the present invention is the biological information estimation device according to the first aspect of the invention, wherein the estimation means computes the volume of the liquid contained in the tissue from the following equation.

[Mathematical 1]

$$V = \frac{M}{\sigma E^2} SAR$$

where V is the volume of the liquid contained in the tissue, M is the mass of the tissue, $\sigma$ is the conductivity of the tissue, E is the electric field strength of the radio waves received by the radio wave receiving means, and SAR is the specific absorption rate of the tissue.

In the second aspect, the above estimating equation which does not make such an assumption that the tissue is spherical is used. By so doing, the volumes of liquids contained in various tissues can be estimated without relying on the shape of the tissue.

A third aspect of the present invention is the biological information estimation device according to the first aspect of the invention, wherein the estimation means estimates the amount of change in the volume of the liquid contained in the tissue based on the amount of change in the amplitude or phase of the radio waves received by the radio wave receiving means, and based on the specific absorption rate.

In the third aspect, the amount of change in the volume of the liquid contained in the tissue can be estimated based on the specific absorption rate of the tissue and the amount of change in the amplitude or phase of the radio waves.

A fourth aspect of the present invention is the biological information estimation device according to the third aspect of the invention, wherein the estimation means computes the amount of change in the volume of the liquid contained in the tissue from the following equation.

[Mathematical 2]

$$\Delta V = \frac{M}{\sigma} SAR \left( \frac{1}{E_{max}^2} - \frac{1}{E_{min}^2} \right)$$

where $\Delta V$ is the amount of change in the volume of the liquid contained in the tissue, M is the mass of the tissue, $\sigma$ is the conductivity of the tissue, $E_{max}$ and $E_{min}$ are the maximum value and the minimum value, respectively, of the electric field strength of the radio waves received by the radio wave receiving means, and SAR is the specific absorption rate of the tissue.

In the fourth aspect, the above estimating equation which does not make such an assumption that the tissue is spherical is used. By so doing, the amounts of change in the volumes of liquids contained in various tissues can be estimated without relying on the shape of the tissue.

A fifth aspect of the present invention is the biological information estimation device according to the third aspect of the invention, wherein the estimation means estimates the amount of change in the volume of the liquid contained in the tissue based on an increase or a decrease in a drift component contained in the amplitude of the radio waves received by the radio wave receiving means, and estimates the volume of the liquid contained in the tissue based on a direct current component remaining after excluding the drift component from the amplitude.

In the fifth aspect, the volume of water contained essentially in the tissue, and the amount of long-term change in water contained in the tissue can be acquired, and such a volume or such an amount of change can be utilized for the diagnosis of the target person.

A sixth aspect of the present invention is the biological information estimation device according to any one of the first to fifth aspects of the invention, wherein the tissue is the heart or blood vessel, and the liquid contained in the tissue is blood.

In the sixth aspect, the volume of blood contained in the heart, or the amount of its change (stroke volume), and further the heart volume can be estimated.

A seventh aspect of the present invention is the biological information estimation device according to any one of the first to fifth aspects of the invention, wherein the tissue is the lung, and the liquid contained in the tissue is water.

In the seventh aspect, the amount of change in water within the lung can be estimated.

An eighth aspect of the present invention is the biological information estimation device according to any one of the first to fifth aspects of the invention, wherein the tissue is the urinary bladder, and the liquid contained in the tissue is urine.

In the eighth aspect, the amount of change in urine within the urinary bladder can be estimated.

A ninth aspect of the present invention is the biological information estimation device according to any one of the first to fifth aspects of the invention, wherein the tissue is the upper or lower limb, and the liquid contained in the tissue is water.

In the ninth aspect, the amount of change in water accumulated in the upper or lower limb by edema can be estimated.

**Effects of the Invention**

According to the present invention, there is provided a biological information estimation device which can estimate the volume of a liquid contained in a tissue, or the amount of change in the volume, under non-contact and non-restraint conditions.

**Brief Description of the Drawings**

[Fig. 1] is a schematic block diagram of a biological information estimation device according to Embodiment 1.
[Figs. 2(a), 2(b)] are schematic views showing the arrangement of antennas relative to the heart.
[Fig. 3] is a view showing electric field strength (simply, field strength) outputted by a sampling unit.
[Fig. 4] is a view showing the field strength outputted by the sampling unit.
[Fig. 5] is a schematic view for explaining a mechanism by which the amount of blood flowing through a blood vessel is estimated.
[Fig. 6] is a schematic view for explaining a mechanism by which the amount of blood flowing through a blood vessel is estimated.
[Fig. 7] is a schematic view for explaining a mechanism by which the amount of blood flowing through a blood vessel is estimated.
[Figs. 8(a), 8(b)] are schematic views showing the arrangement of antennas relative to the lung.
[Fig. 9] is a view showing the field strength outputted by the sampling unit.
[Fig. 10] is a view showing the field strength outputted by the sampling unit when microwaves of a pulse waveform are used.

**Mode for Carrying Out the Invention**

<Embodiment 1>

A biological information estimation device according to the present invention (hereinafter referred to simply as an estimation device) is a device which is targeted at a tissue of a living body for estimating biological information under non-contact and non-restraint conditions. The biological information refers to the volume of a liquid contained in the tissue of the living body, or the amount of change in the volume. The tissue of the living body is exemplified by, but not limited to, the heart, blood vessel, lung, upper or lower limb, or bladder, and the present invention can be applied to any tissue with a known specific absorption rate. The liquid contained in the tissue is blood in the case of the heart and blood vessel, water in the case of the lung or upper or lower limb, and urine in the case of the bladder.

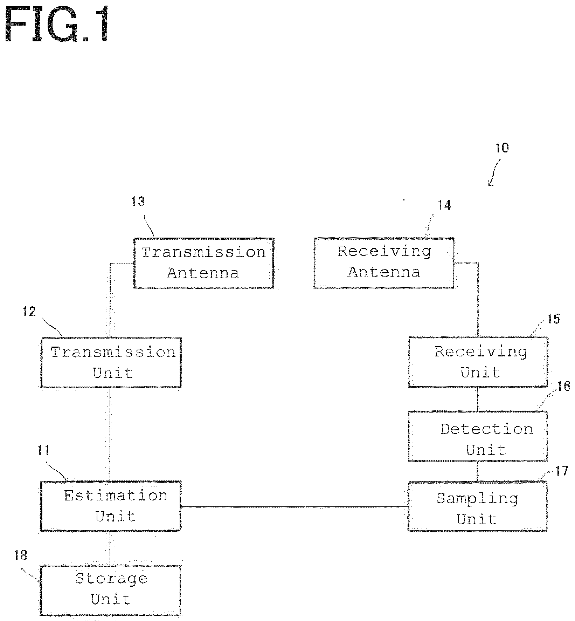

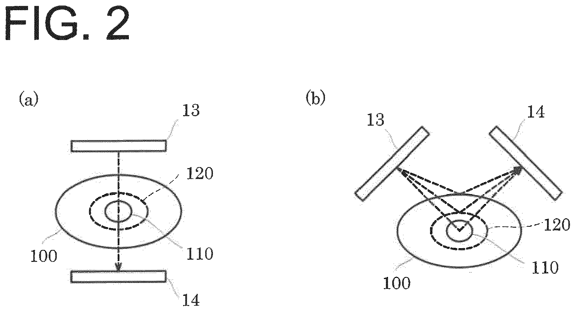

In the present embodiment, an explanation will be offered for an estimation device which is targeted at the heart of a human body as a tissue of a living body and estimates the volume of blood contained in the heart. Fig. 1 is a schematic block diagram of the biological information estimation device according to the present embodiment. Figs. 2(a) and 2(b) are schematic views showing the arrangement of antennas relative to the heart. The numeral 110 in Figs. 2(a), 2(b) denotes the heart in the systolic phase, and the numeral 120 denotes the heart in the diastolic phase.

As shown in Fig. 1, an estimation device 10 is equipped with an estimation unit 11 as an estimation means, a transmission unit 12 and a transmission antenna 13 as a radio wave transmission means, a receiving unit 15 and a receiving antenna 14 as a radio wave receiving means, a detection unit 16, a sampling unit 17, and a storage unit 18.

The transmission unit 12 is a device for transmitting high frequency waves, preferably microwaves, to the human body. The microwaves in any frequency band may be used, as long as they can pass through the heart of the human body, or can be reflected by the heart. In the present embodiment, a frequency of around 1 GHz including a sub-gigahertz band is used. Transmission output may be such that sufficient electric power can be detected on the receiving side. In the present embodiment, the transmission output is set at several mW to several tens of mW. The microwaves may be continuous waves, pulse waves, or phase-modulated or frequency-modulated electromagnetic waves. The transmission unit 12 supplies high frequency signals, which have been generated by a microwave oscillator (not shown), to the transmission antenna 13.

The transmission antenna 13 is an instrument for emitting the microwaves, which have been transmitted by the transmission unit 12, toward the heart of a human body 100. The receiving antenna 14 is an instrument for receiving the microwaves radiated from the transmission antenna 13.

As shown in Fig. 2(a), the transmission antenna 13 and the receiving antenna 14 are installed opposite each other so that the microwaves pass through the heart 110 of the human body 100. As polarized waves, either horizontally polarized waves or vertically polarized waves may be used. Alternatively, as shown in Fig. 2(b), the transmission antenna 13 may be disposed so as to output microwaves toward the heart 110, while the receiving antenna 14 may be disposed so as to receive the microwaves reflected by the heart 110. The microwaves either pass through the human body, or are reflected at different locations of the human body, whereupon the microwaves are changed in amplitude or phase and received by the receiving antenna 14.

In the example of Fig. 2(a), the transmission antenna 13 is installed ahead of the human body 100, and the receiving antenna 14 is installed behind the human body 100. However, their positions may be reversed, or these antennas may be arranged on both sides of the human body 100. Anyway, the antennas may be arranged at such positions that the microwaves can pass through the human body 100 and can be received. In the example of Fig. 2(b), the transmission antenna 13 and the receiving antenna 14 are arranged forward of the human body 100, but there is no limitation on their arrangement. Moreover, dipole antennas are used as the transmission antenna 13 and the receiving antenna 14, but the type of the antenna is not particularly limited. As regards polarized waves, either horizontally polarized waves or vertically polarized waves may be used.

As shown in Fig. 1, the receiving unit 15 is a means of converting signals of the transmitted microwaves, which have been received by the receiving antenna 14, into signals to be needed by the detection unit 16. The detection unit 16 is a means of detecting the microwaves received by the receiving unit 15. The detection unit 16 demodulates the microwaves by envelope detection (amplitude detection) or phase detection. The detection unit 16 may also take out a specific frequency component by frequency analysis to demodulate the microwaves.

The sampling unit 17 is a means of sampling the detected signals with the use of a preset frequency to convert the field strength into digital signals. Concretely, sampling is performed by a publicly known A/D converter, or by processing using software.

The storage unit 18 is a device, such as a memory or a hard disk, which functions as a storage area necessary for various computations to be carried out in the estimation unit 11. The storage unit 18 stores an estimating equation and various parameters, such as the specific absorption rate, conductivity, and mass of the heart, which will be described later.

The estimation unit 11 is a means which, after instructing the transmission unit 12 to output microwaves, analyzes the amplitude and phase of the radio waves represented as digital signals that have been received from the sampling unit 17, and computes the volume of blood contained in the heart. The thus computed blood volume is estimated as the volume of blood contained in the heart of the human body 100.

In the present embodiment, the estimation unit 11 is mounted as the function of a program to be executed by an information processor such as a general personal computer. The transmission unit 12, receiving unit 15, detection unit 16 and sampling unit 17 are mounted as an electronic circuit (hardware) and can be controlled by the estimation unit 11. It goes without saying that each of the estimation unit 11, transmission unit 12, receiving unit 15, detection unit 16, and sampling unit 17 may be mounted using a program, or they may be mounted as an electronic circuit.

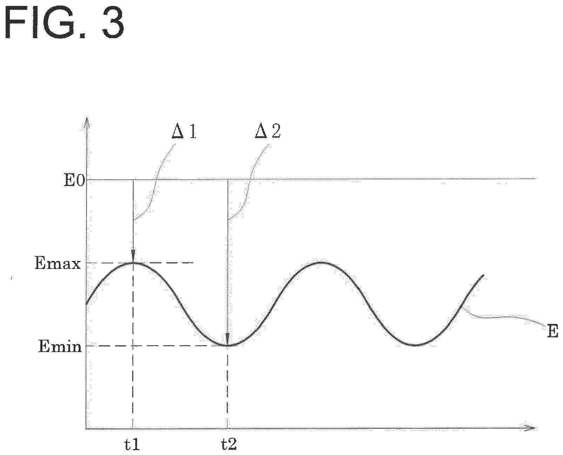

Fig. 3 is a view showing radio waves outputted by the sampling unit 17. The abscissa axis of Fig. 3 represents time, whereas the ordinate axis of Fig. 3 represents field strength (amplitude).

The symbol E0 in the drawing shows the field strength of the microwaves emitted by the transmission antenna 13, and this field strength remains constant. The symbol E denotes the field strength E which has been received by the receiving antenna 14, detected by the detection unit 16, and digitized by the sampling unit 17. The time when the heart is in the systolic phase is designated as t1, while the time when the heart is in the diastolic phase is designated as t2. Of the values of the field strength E, the field strength at the time t1 is called $E_{max}$, and the field strength at the time t2 is called $E_{min}$.

The field strength E of the microwaves that have passed through, or have been reflected by, the heart changes in strength (decays) mainly because of the blood volume within the heart. When the blood volume is small, i.e., in the systolic phase of the heart (time t1), for example, the amount of decay, $\Delta 1$, of the field strength is relatively small. When the blood volume is large, i.e., during diastole of the heart (time t2), on the other hand, the amount of decay, $\Delta 2$, of the field strength is relatively large.

As described above, the field strength E changes in amplitude in conformity with the contraction and expansion of the heart. Changes in the field strength E are considered to be information related closely to the contraction and expansion of the heart. By analyzing the field strength, therefore, the volume of blood contained in the heart can be estimated. Here, the blood contained in the heart refers to blood in the ventricles and atria. The volume of the blood in the ventricles and atria is nearly equal to the volume of the atria and ventricles (heart volume). Thus, the estimation of the volume of the blood contained in the heart is synonymous with the estimation of the heart volume.

The volume of the blood contained in the heart is computed using the field strength E obtained as mentioned above, the specific absorption rate of the heart, and an estimating equation. The specific absorption rate (SAR) refers to the amount of energy absorbed per unit time into a tissue of a unit mass of a human body, as shown in Equation 1, and its unit is [W/kg].

[Mathematical 3]

$$\text{SAR} = \frac{\sigma E^2}{\rho} \quad \text{(Equation 1)}$$

In the above equation, $\sigma$ represents the conductivity [S/m] of the heart, and $\rho$ represents the density [kg/m$^3$] of the heart. The specific absorption rate is determined as in Equation 1. Since the specific absorption rate of the heart is publicly known, it is stored in the storage unit 18 beforehand.

The density $\rho$ of Equation 1 is defined as in Equation 2. In this equation, V represents the volume [m$^3$] of the heart, and M represents the mass [kg] of the heart.

[Mathematical 4]

$$\rho = \frac{M}{V} \quad \text{(Equation 2)}$$

Substituting Equation 2 into Equation 1, followed by rearranging the terms for V, obtains Equation 3. This Equation 3 is the aforementioned estimating equation.

[Mathematical 5]

$$V = \frac{M}{\sigma E^2} SAR \quad \text{(Equation 3)}$$

The mass M, conductivity $\sigma$, and specific absorption rate SAR of the heart used are publicly known ones, and are stored beforehand in the storage unit 18. The estimation unit 11 reads the mass M, conductivity $\sigma$, and specific absorption rate SAR from the storage unit 18, substitutes them, together with the field strength E obtained from the sampling unit 17, into the above estimating equation (Equation 3) to obtain V, and deems the resulting V as the volume of blood contained in the heart, namely, the heart volume.

According to the estimation device 10, as described above, microwaves which have passed through, or have been reflected by, the human body are used. Thus, the volume of blood in the heart and the heart volume can be estimated, without contact with the human body and without restraint of the human body. The estimation device 10, moreover, uses the conductivity and specific absorption rate of the heart, but makes no assumption about the shape of the heart. Hence, the volume of blood contained in the heart and the heart volume can be estimated, with higher accuracy than provided on the assumption, for example, that the shape of the heart is spherical. Since an assumption about shape, such that the heart is spherical, is not made, moreover, the volume of a liquid contained in a tissue, even one other than the heart, can be estimated.

<Embodiment 2>

The estimation device 10 of Embodiment 1 estimates the volume of blood contained in the heart and the heart volume, but is not limited to these parameters. For example, the estimation device 10 may estimate the amount of change in each of the volume of blood and the heart volume. In the present embodiment, an explanation will be offered for an estimation device 10 which estimates such an amount. The estimation device 10 of the present embodiment has the same configuration as that of the estimation device 10 of Embodiment 1, and thus its illustration will be omitted.

As shown in Fig. 3, the field strength E changes according to the contraction and expansion of the heart. The difference between the field strengths at any two timings, therefore, can be estimated to be an amount correlated with a difference in the volume of blood in the heart. As the two timings, the systolic phase and the diastolic phase of the heart are selected. By so doing, the amount of change between the volumes of blood in the systolic phase and the diastolic phase can be estimated. This amount of change in the blood volume is considered to correspond to a stroke volume. The stroke volume refers to the amount of blood ejected from the ventricle per beat.

Based on the above mechanism, an estimation unit 11 of the present embodiment computes the amount of change in the volume of blood in the heart from a change in the amplitude of the field strength E and from the specific absorption rate. Concretely, the estimation unit 11 specifies each of the field strength $E_{max}$ in the systolic phase and the field strength $E_{min}$ in the diastolic phase. For example, during the cycle lasting for single ejection of the heart, the highest field strength is detected, and taken as the field strength $E_{max}$ in the systolic phase. Similarly, the lowest field strength is detected, and taken as the field strength $E_{min}$ in the diastolic phase.

These field strengths $E_{max}$ and $E_{min}$ are applied to the aforementioned estimating equation (Equation 3) to find the volume of blood contained in the heart in the systolic phase, $V_s$, (heart volume $V_s$) and the volume of blood contained in the heart in the diastolic phase, $V_d$, (heart volume $V_d$). Then, the difference between the heart volume $V_s$ and the heart volume $V_d$ is calculated, as shown in Equation 4.

[Mathematical 6]

$$\Delta V = V_d - V_s$$

$$\Delta V = \frac{M}{\sigma E_{max}^2} SAR - \frac{M}{\sigma E_{min}^2} SAR$$

$$\Delta V = \frac{M}{\sigma} SAR \left( \frac{1}{E_{max}^2} - \frac{1}{E_{min}^2} \right) \qquad \text{(Equation 4)}$$

The amount of change $\Delta V$ in the heart volume, which is the difference between the heart volume $V_s$ and the heart volume $V_d$, represents a stroke volume which is the amount [mL] of blood ejected to the arteries per beat of the heart. By so applying the amount of change in the field strength E and the specific absorption rate of the heart to the estimating equation, it becomes possible to obtain the amount of change $\Delta V$ in the heart volume, and the stroke volume which is the amount of change in blood contained in the heart.

In the example shown in Fig. 3, the amount of change in the heart volume is found based on the difference in the field strength received, namely, the difference in the amplitude. However, such an embodiment is not limitative, and the amount of change in the heart volume may be found based on the difference in phase.

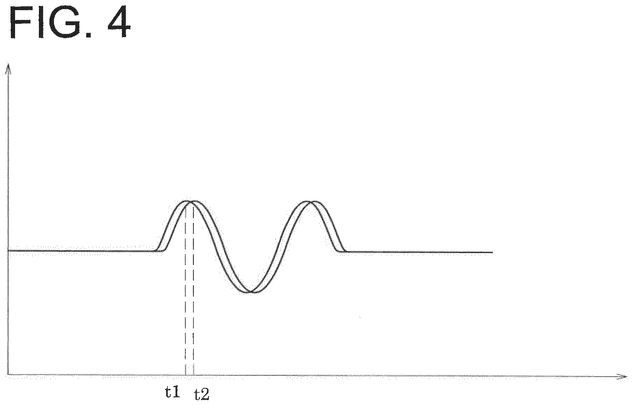

Fig. 4 is a view showing the field strength outputted by the sampling unit. The abscissa axis of Fig. 4 represents time, whereas the ordinate axis of Fig. 4 represents field strength (amplitude). Microwaves of a pulse waveform are reflected by the human body, and the reflected waves are detected by the sampling unit 17 to be illustrated here. The microwaves are observed, with the phase of the pulse waves being shifted. As illustrated in the drawing, a phase difference which is a shift in phase by a time difference between the time t1 and the time t2, for example, is observed. Such a phase difference of radio waves is considered to result from the contraction and expansion of the heart.

The phase difference of radio waves, therefore, can be estimated to be an amount correlated with a difference in the volume of blood in the heart. Thus, the estimation unit 11 can estimate the amount of change in the volume of blood by calculating the phase difference of the resulting radio waves, and subjecting the calculated phase difference to a predetermined calculation.

<Embodiment 3>

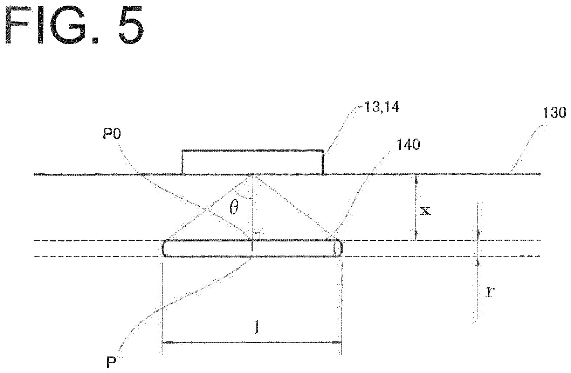

An estimation device 10 of the present embodiment targets blood flowing through a blood vessel, as a liquid contained in a tissue. The estimation device 10 of the present embodiment has the same configuration as that of the estimation device 10 of Embodiment 1, and thus its illustration will be omitted. Fig. 5 is a schematic view for explaining a mechanism by which to estimate the flow rate of blood flowing through the blood vessel (hereinafter, blood volume).

Inside a skin 130 is a blood vessel 140 and, on the surface side of the skin 130, is disposed an integration of a transmission antenna 13 and a receiving antenna 14 for microwaves. The transmission antenna 13 outputs microwaves toward the skin 120 at a predetermined irradiation angle θ. The receiving antenna 14 receives the microwaves reflected by the blood vessel 140.

If the blood vessel 140 is targeted, as above, it is conceivable that the microwaves will pass through the interior of the blood vessel 140 and be absorbed by blood, as in the case of the heart. Since the blood vessel 140 pulsates and changes in diameter, the blood volume also fluctuates. If the blood volume fluctuates, the amount of the microwaves absorbed also fluctuates. Thus, a change in the field strength received by the receiving antenna 14 can be grasped as a change in the blood volume.

A concrete method of estimation will be described. Let the length, in a predetermined range, of a single blood vessel 140 as an estimation target be 1. Then, the length 1 [m] is defined by Equation 5.

[Mathematical 7]

$$l = 2x \tan\theta \quad \text{(Equation 5)}$$

where x represents the distance from the surface of the skin 130 to the blood vessel 140, and θ represents the irradiation angle of the microwaves. Then, if the diameter of the single blood vessel 140 is designated as r, the blood vessel diameter r is defined by Equation 6.

[Mathematical 8]

$$r = -\frac{1}{2\gamma} \log\left(\frac{V}{V_0}\right) \quad \text{(Equation 6)}$$

$$\gamma = \sqrt{\pi f \mu \sigma}$$

$$\text{SAR} = \text{SAR}_0 \cdot e^{-2\gamma r}$$

$$E = \frac{V}{l}$$

ρ is the density [kg/m$^3$] of the interior of a biological tissue (blood vessel), E is the effective value [V/m] of an electric field obtained by the receiving antenna 14, $\text{SAR}_0$ is the specific absorption rate of the blood vessel at a reference position P0, SAR is the specific absorption rate of the blood vessel at a position advanced by the blood vessel diameter r from the reference position P0 toward the inside of the living body, f is the frequency [Hz] of the microwaves, μ is the magnetic permeability [H/m] of the blood vessel, σ is the conductivity [S/m] of the blood vessel, V is the induced voltage [V] of the receiving antenna 14, and $V_0$ is a voltage [V] outputted by the transmission antenna 13.

Let the volume, in a predetermined range, of the single blood vessel 140 as the estimation target be Q. Then, the volume Q is defined by Equation 7. The volume (capacity) Q of the blood vessel 140 is synonymous with the volume of blood (blood volume) flowing through the interior of the blood vessel.

[Mathematical 9]

$$Q = \frac{\pi r^2}{4} l \quad \text{(Equation 7)}$$

$$Q = \frac{\pi x \tan\theta}{8\gamma^2} \left\{ \log\left(\frac{V}{V_0}\right) \right\}^2 \quad \text{(Equation 8)}$$

Equations 5 and 6 are substituted, respectively, into the length 1 and the blood vessel diameter r of Equation 7, whereby Equation 8 can be obtained.

A storage unit 18 has the density p, specific absorption rate $\text{SAR}_0$ and specific absorption rate SAR of the blood vessel, the magnetic permeability μ of the blood vessel, and the conductivity σ of the blood vessel stored therein beforehand. An estimation unit 11 substitutes the values read from the storage unit 18, the induced voltage V of radio waves obtained from the receiving antenna 14, the voltage $V_0$ outputted from the transmission antenna 13, and the irradiation angle $\theta$ and frequency f of the microwaves into Equations 6 and 8 to calculate the volume Q.

According to the estimation device 10, as described above, the microwaves reflected by the blood vessel of the human body are used. Thus, the blood volume in the blood vessel can be estimated, without restraint of the human body.

<Embodiment 4>

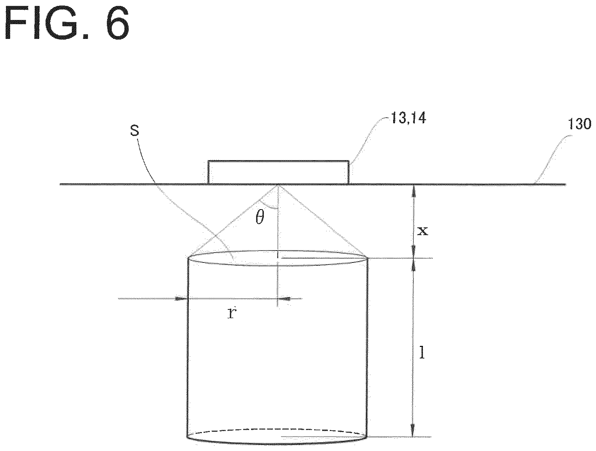

Embodiment 3 assumes a single blood vessel as the target for measurement. However, such an embodiment is not limitative, and the present invention is applicable to even another model. An estimation device 10 of the present embodiment has the same configuration as that of the estimation device 10 of Embodiment 3, and thus its illustration will be omitted. Fig. 6 is a schematic view for explaining a mechanism by which to estimate the amount of blood flowing through a blood vessel. Unless redefined in the present embodiment, variates having the same meanings as those in Embodiment 3 are expressed by using the same parameter names.

The arrangement of a transmission antenna 13 and a receiving antenna 14, and so on are the same as those in Embodiment 3. By changing the directivity and transmission field strength of the transmission antenna 13, it can be expected that the state of absorption of radio waves by a living body will differ. In the present embodiment, therefore, the radiation range of radio waves is assumed to be columnar, and the volume of blood in the entire tissue within this column is estimated.

A concrete method of estimation will be described. A hypothetical circle is assumed on the surface on the skin side of a blood vessel layer as a measurement target. The area S of this circle is defined as in Equation 9. r represents the radius of the hypothetical circle.

[Mathematical 10]

$$S = \pi(xtan\theta)^2 \quad \text{(Equation 9)}$$

$$r = x \tan\theta$$

Let the height of the column be 1. The height 1 of this column represents a depth which can be measured upon irradiation with microwaves. The height 1 is defined as in Equation 10. Equation 10 can be derived as is Equation 6.

[Mathematical 11]

$$l = -\frac{1}{2\gamma} \log\left(\frac{V}{V_0}\right) \quad \text{(Equation 10)}$$

Let the volume of the entire tissue having the volume of the column as the estimation target be Q. Then, the volume Q is defined by Equation 12 which has been derived by substituting Equations 9 and 10 into Equation 11. The volume (capacity) Q of the column is synonymous with the volume of blood (blood volume) flowing through the interior of the column.

[Mathematical 12]

$$Q = S \times l \quad \text{(Equation 11)}$$

$$Q = -\frac{\pi(x \tan\theta)^2}{2\gamma} \log\left(\frac{V}{V_0}\right) \quad \text{(Equation 12)}$$

A storage unit 18 has the density p, specific absorption rate $SAR_0$ and specific absorption rate SAR of the blood vessel, the magnetic permeability $\mu$ of the blood vessel, and the conductivity $\sigma$ of the blood vessel stored therein beforehand. An estimation unit 11 substitutes the values read from the storage unit 18, the induced voltage V of radio waves obtained from the receiving antenna 14, the voltage $V_0$ outputted from the transmission antenna 13, and the irradiation angle $\theta$ and frequency f of the microwaves into Equation 12 to calculate the volume Q. The volume Q means the volume of blood flowing in the columnar region which is the radiation range of the microwaves.

According to the estimation device 10, as described above, the microwaves reflected by the blood vessel of the human body are used. Thus, the blood volume in the blood vessel can be estimated, without restraint of the human body. In particular, a model based on a single blood vessel as in Embodiment 3 is not assumed, but the radiation range of the microwaves is interpreted as a column. By so doing, if the range of measurement is spread by the directivity of the transmission antenna 13, the blood volume can be estimated with particularly high accuracy.

<Embodiment 5>

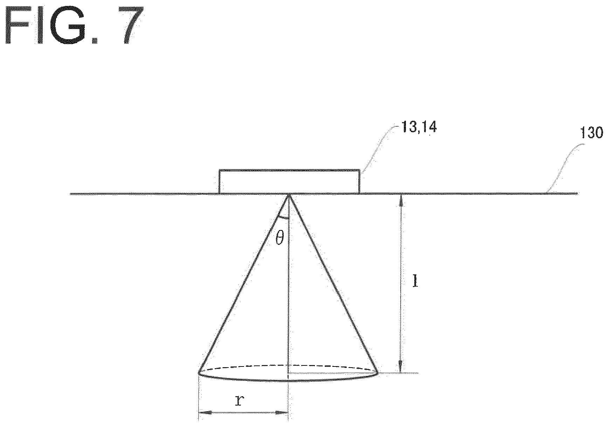

Embodiment 4 assumes a columnar range, in which blood is flowing, as the target for measurement. However, such an embodiment is not limitative, and the present invention is applicable to even a different model. An estimation device 10 of the present embodiment has the same configuration as that of the estimation device 10 of Embodiment 3, and thus its illustration will be omitted. Fig. 7 is a schematic view for explaining a mechanism by which to estimate the amount of blood flowing through a blood vessel. Unless redefined in the present embodiment, variates having the same meanings as those in Embodiment 4 are expressed by using the same parameter names.

The arrangement of a transmission antenna 13 and a receiving antenna 14, and so on are the same as those in Embodiment 4. By changing the directivity and transmission field strength of the transmission antenna 13, it can be expected that the state of absorption of radio waves by a living body will differ. In the present embodiment, the radiation range of radio waves is assumed to be conical, and the amount of a blood flow in all of tissues within this cone is estimated.

A concrete method of estimation will be described. Assume a cone which has a vertex at a portion of the transmission antenna 13 emitting microwaves and which extends inside a skin 130. Let the radius of a circle at the base of this cone be r, and the height of the cone (depth from the skin 130) be 1. Then, the area S of the base of the cone is defined as in Equation 13. r is the radius of the hypothetical circle.

[Mathematical 13]

$$S = \pi(l \tan \theta)^2 \quad \text{(Equation 13)}$$

$$r = l \tan \theta$$

The height 1 of the cone is defined as in Equation 14. Equation 14 can be derived as is Equation 6.

[Mathematical 14]

$$l = -\frac{1}{2\gamma} \log\left(\frac{V}{V_0}\right) \quad \text{(Equation 14)}$$

Let the volume of the entire tissue having the volume of the cone as the estimation target be Q. Then, the volume Q is defined by Equation 16 which has been derived by substituting Equations 13 and 14 into Equation 15. The volume (capacity) Q of the cone is synonymous with the volume of blood (blood volume) flowing through the interior of the cone.

[Mathematical 15]

$$Q = \frac{1}{3}\pi r^2 \times l \quad \text{(Equation 15)}$$

$$Q = -\frac{\pi(\tan \theta)^2}{24\gamma}\left\{\log\left(\frac{V}{V_0}\right)\right\}^3 \quad \text{(Equation 16)}$$

A storage unit 18 has the density p, specific absorption rate $SAR_0$ and specific absorption rate SAR of the blood vessel, the magnetic permeability $\mu$ of the blood vessel, and the conductivity $\sigma$ of the blood vessel stored therein beforehand. An estimation unit 11 substitutes the values read from the storage unit 18, the induced voltage V of radio waves obtained from the receiving antenna 14, the voltage $V_0$ outputted from the transmission antenna 13, and the irradiation angle $\theta$ and frequency f of the microwaves into Equation 16 to calculate the volume Q. The volume Q means the volume of blood flowing in the conical region which is the radiation range of the microwaves.

According to the estimation device 10, as described above, the microwaves reflected by the blood vessel of the human body are used. Thus, the blood volume in the blood vessel can be estimated, without restraint of the human body. In particular, a blood vessel layer at a position sufficiently close to the surface of the skin 130 is targeted. Hence, the estimation device 10 is useful in a case where the distance from the skin 130 to the blood vessel layer is negligible.

<Embodiment 6>

The estimation devices 10 described in Embodiments 1 and 2 target the heart or blood vessel, but this is not limitative. The present invention utilizes the decay of field strength due to a liquid. Hence, the present invention does not rely on a change in the shape of a tissue, but is applicable even to the essential volume of the liquid within the tissue, or to the amount of change in the volume. The estimation device 10 can measure, for example, the amount of change in water accumulated in the lung because of pulmonary congestion. An estimation device 10 of the present embodiment has the same configuration as that of the estimation device 10 of Embodiment 1, and thus its illustration will be omitted.

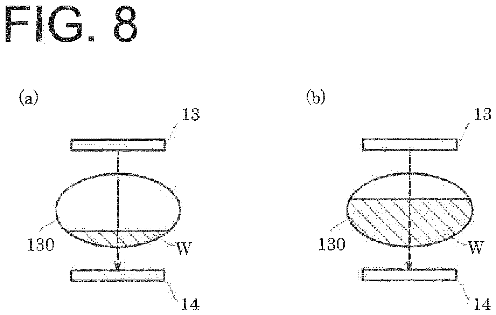

Figs. 8(a), 8(b) are schematic views showing the arrangement of antennas with respect to the lung. Fig. 8(a) shows a state in which the amount of water W within the lung is small, whereas Fig. 8(b) shows a state in which the amount of water W within the lung is large. As shown in these drawings, a transmission antenna 13 and a receiving antenna 14 are arranged so as to emit microwaves toward the lung and receive the microwaves that have passed through the lung. As revealed in Fig. 2(b), the transmission antenna 13 and the receiving antenna 14 may be arranged so as to reflect microwaves by the lung and receive the microwaves, although this arrangement is not shown in the drawings.

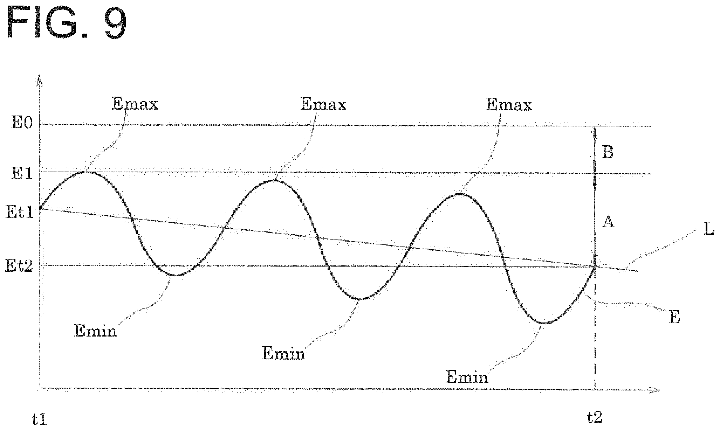

Fig. 9 is a view showing field strengths outputted from a sampling unit 17. The abscissa axis of Fig. 9 represents time, whereas the ordinate axis of Fig. 9 represents field strength. A symbol E0 in the drawing shows the field strength of microwaves emitted by the transmission antenna 13, and this field strength remains constant. A symbol E denotes the field strength E of the microwaves which have been received by the receiving antenna 14, detected by a detection unit 16, and digitized by the sampling unit 17. A symbol E1 represents a straight line of field strength reaching the highest value of the field strength E and parallel to the line of E0. The time when the amount of water is small as shown in Fig. 8(a) is designated as t1, while the time when the amount of water is large as shown in Fig. 8(b) is designated as t2.

The field strength E of the microwaves that have passed through, or have been reflected by, the lung decays owing to the water within the lung. The field strength E of the microwaves periodically changes as the lung expands and contracts. In the long term, when the amount of water is small (time t1) as shown in Fig. 8(a), the amount of the microwaves absorbed by water is small, so that the field strength E is observed to be relatively high. When the amount of water is large (time t2) as shown in Fig. 8(b), on the other hand, the field strength E is observed to be relatively low.

The microwaves decay from the field strength E0 to the field strength E. This amount of decay consists of a drift component A and a direct current component B. The drift component A is a portion corresponding to the difference between the field strength E and the field strength E1 of the amount of decay. The drift component B is a portion corresponding to the difference between the field strength E0 and the field strength E1.

From the drift component A of the microwaves, a long-term increase or decrease in the amount of water can be estimated. For example, the time t1 and the time t2 are selected so that the field strength Et1 and the field strength Et2 will have the same phase, and the two field strengths Et1 and Et2 are connected together to form a straight line L. This straight line L represents the tendency of the drift component A.

If the slope of such a straight line L is negative, that is, if the drift component A decreases over time, the amount of water in the lung increases. In contrast, the slope of the straight line L being positive, namely, the drift component A increasing over time, means the water content of the lung on the decrease. Based on the increase or decrease in the drift component A, therefore, the amount of long-term change in the volume of water contained in the lung can be estimated.

The direct current component B, on the other hand, is considered to be a portion of the microwaves decayed by an unchanged part of the water contained in the lung. In other words, the microwaves are decayed by the water essentially contained in the lung, and the amount of the decay corresponds to the direct current component B. Hence, the volume of the water essentially contained in the lung can be estimated based on the direct current component of the microwaves.

As noted above, it is possible to obtain, from the microwaves, the volume of water essentially contained in the lung based on the direct current component B, the amount of short-term change in water contained in the lung based on the amount of change in the amplitude (or phase) of the microwaves during one cycle, and the amount of long-term change in water contained in the lung based on the amount of change in the drift component A.

As described in Embodiment 1, it is also possible to estimate the amount of short-term change in water contained in the lung based on the difference between the strengths $E_{max}$ and $E_{min}$ of the microwaves during any one cycle.

Based on such mechanisms, the estimation device 10 estimates the volume of water contained in the lung, and the amount of change in the volume of the water, in the following manner: The mass M, conductivity $\sigma$, and specific

absorption rate SAR of the lung are stored beforehand in a storage unit 18. In an estimation unit 11, the field strength E obtained from the sampling unit 17, and the mass M, conductivity $\sigma$, and specific absorption rate SAR read from the storage unit 18 are substituted into the aforementioned estimating equation (Equation 3), whereby the volume V of the lung can be found. This volume V of the lung is estimated to be the amount of water contained in the lung.

The estimation unit 11 calculates the amount of change $\Delta V$ in the volume of the lung from Equation 4, using the minimum value $E_{min}$ and the maximum value $E_{max}$ of the field strength E, for example, per cycle of respiration of the lung. This amount of change $\Delta V$ is estimated to be the amount of change $\Delta V$ in the water contained in the lung.

The estimation unit 11 also finds the drift component A at each of any two times t1 and t2 with a sufficient time interval being provided therebetween. If the drift component A decreases over time, the water contained in the lung is estimated to be increasing. If the drift component A increases over time, the water contained in the lung is estimated to be decreasing.

The estimation unit 11, moreover, computes the direct current component B of the microwaves and, based on the direct current component B, estimates the volume of the water contained essentially in the lung. For example, the estimation unit 11 estimates the volume of the water, for example, by multiplying the direct current component B by a predetermined coefficient.

In the above-mentioned example, the rate of increase or decrease in the water contained in the lung is slow, and thus measurement needs to be made over a long term, for several hours. In such a case, it is preferred to use microwaves of a pulse waveform, instead of emitting microwaves continuously.

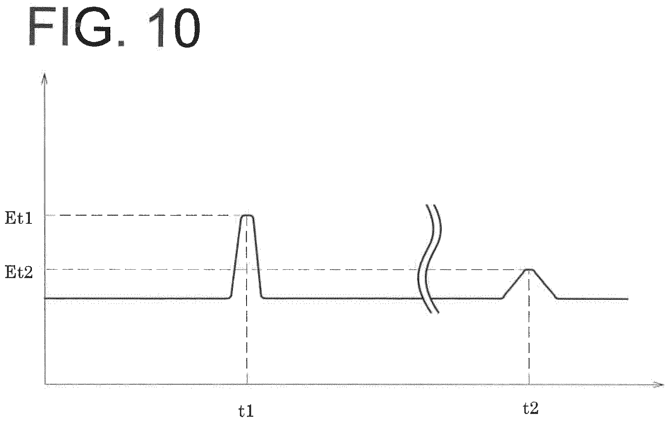

Fig. 10 is a view showing the field strength of microwaves outputted by the sampling unit when the microwaves of a pulse waveform are used.

Concretely, the transmission antenna 13 emits microwaves to an organ or the like of a living body in a pulse waveform at an arbitrary cycle. The receiving antenna 14 receives the microwaves which have passed through, or have been reflected by, the organ. The so obtained field strength E is in a pulse waveform.

When the amount of water is small (time t1) as shown in Fig. 8(a), the amount of the microwaves absorbed by water is small, so that the field strength Et1 is observed to be relatively high. When the amount of water is large (time t2) as shown in Fig. 8(b), on the other hand, the field strength Et2 is observed to be relatively low. In short, in a case where the water in the lung is gradually increasing, the field strength tends to lower in the long term. In a case where the water in the lung is gradually decreasing, the field strength tends to rise in the long term, although this is not shown.

As described above, even when the pulse waveform is used, the volume V of the lung and the amount of its change, $\Delta V$, can be found. Since there is no need to constantly emit microwaves to the living body, moreover, the influence of the microwaves on the living body can be suppressed, and power consumption can be reduced.

The estimation device 10 of the present embodiment estimates the amount of change in water in the lung caused by pulmonary congestion, but this aspect is not limitative. For example, the amount of change in the volume of urine accumulated in the bladder can be estimated similarly. That is, if the amount of change in the amplitude of microwaves obtained by emitting the microwaves to the bladder tends to decrease as in Fig. 9, it can be estimated that urine accumulated in the bladder is on the increase.

Furthermore, the amount of change in the volume of water accumulated in the limb due to edema can also be estimated in the same manner as in the present embodiment. That is, if the amount of change in the amplitude of microwaves obtained upon emission of the microwaves to the limb tends to decrease as in Fig. 9, it can be estimated that water accumulated in the limb is on the increase. Such microwaves of a pulse waveform can be targeted not only at the lung, but can also be applied to other tissues such as the heart, blood vessel, bladder or limbs.

As described above, the estimation device 10 according to the present embodiment emits microwaves to various tissues, such as the lung, bladder and limbs, and receives the waves reflected by or passed through the tissue, thereby becoming capable of estimating the volume of water contained in the tissue, or the amount of change in the volume. Concretely, the estimation device 10 can grasp the volume of water essentially contained in the tissue, as well as the amounts of long-term and short-term changes in water contained in the tissue, and can utilize the volume of the water, or the amount of change in the volume, for diagnosis of the target person.

**Explanations of Letters or Numerals**

    10 Biological information estimation device
    11 Estimation unit (estimation means)
    12 Transmission unit (radio wave transmission means)
    13 Transmission antenna (radio wave transmission means)
    14 Receiving antenna (radio wave receiving means)
    15 Receiving unit (radio wave receiving means)

16 Detection unit
17 Sampling unit
18 Storage unit (storage means)

**Claims**

1. A biological information estimation device, comprising:

   radio wave transmission means for emitting radio waves toward a tissue of a living body;
   radio wave receiving means for receiving the radio waves which have passed through the tissue, or the radio waves which have been reflected by the tissue; and
   estimation means for estimating a volume of a liquid contained in the tissue based on an amplitude or phase of the radio waves received by the radio wave receiving means, and a specific absorption rate of the tissue.

2. The biological information estimation device according to claim 1, wherein
   the estimation means computes the volume of the liquid contained in the tissue from the following equation:

   [Mathematical 1]

   $$V = \frac{M}{\sigma E^2} SAR$$

   where V is the volume of the liquid contained in the tissue, M is a mass of the tissue, $\sigma$ is a conductivity of the tissue, E is the amplitude of the radio waves received by the radio wave receiving means, and SAR is the specific absorption rate of the tissue.

3. The biological information estimation device according to claim 1, wherein
   the estimation means estimates an amount of change in the volume of the liquid contained in the tissue based on an amount of change in the amplitude or phase of the radio waves received by the radio wave receiving means, and based on the specific absorption rate.

4. The biological information estimation device according to claim 3, wherein
   the estimation means computes the amount of change in the volume of the liquid contained in the tissue from the following equation:

   [Mathematical 2]

   $$\Delta V = \frac{M}{\sigma} SAR \left( \frac{1}{E_{max}^2} - \frac{1}{E_{min}^2} \right)$$

   where $\Delta V$ is the amount of change in the volume of the liquid contained in the tissue, M is a mass of the tissue, $\sigma$ is a conductivity of the tissue, $E_{max}$ and $E_{min}$ are a maximum value and a minimum value, respectively, of the electric field strength of the radio waves received by the radio wave receiving means, and SAR is the specific absorption rate of the tissue.

5. The biological information estimation device according to claim 3, wherein
   the estimation means estimates the amount of change in the volume of the liquid contained in the tissue based on an increase or a decrease in a drift component contained in the amplitude of the radio waves received by the radio wave receiving means, and estimates the volume of the liquid contained in the tissue based on a direct current component remaining after excluding the drift component from the amplitude.

6. The biological information estimation device according to any one of claims 1 to 5, wherein
   the tissue is a heart or a blood vessel, and the liquid contained in the tissue is blood.

7. The biological information estimation device according to any one of claims 1 to 5, wherein the tissue is a lung, and the liquid contained in the tissue is water.

8. The biological information estimation device according to any one of claims 1 to 5, wherein the tissue is a urinary bladder, and the liquid contained in the tissue is urine.

9. The biological information estimation device according to any one of claims 1 to 5, wherein the tissue is an upper limb or a lower limb, and the liquid contained in the tissue is water.

# FIG.1

# FIG. 2

(a)

13

120

100

110

14

(b)

13

14

120

110

100

# FIG. 3

FIG. 4

t1  t2

# FIG. 5

# FIG. 6

# FIG. 7

# FIG. 8

(a)

(b)

FIG. 9

# FIG. 10

## INTERNATIONAL SEARCH REPORT

| International application No. |
| --- |
| PCT/JP2018/016009 |

**A. CLASSIFICATION OF SUBJECT MATTER**
Int.Cl. A61B5/026(2006.01)i, A61B5/00(2006.01)i, A61B5/029(2006.01)i

According to International Patent Classification (IPC) or to both national classification and IPC

**B. FIELDS SEARCHED**

Minimum documentation searched (classification system followed by classification symbols)
Int.Cl. A61B5/026, A61B5/00, A61B5/029

Documentation searched other than minimum documentation to the extent that such documents are included in the fields searched
Published examined utility model applications of Japan       1922–1996
Published unexamined utility model applications of Japan     1971–2018
Registered utility model specifications of Japan             1996–2018
Published registered utility model applications of Japan     1994–2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 3-1838 A (SHIMADZU CORP.) 08 January 1991, entire text, all drawings (Family: none) | 1-9 |
| A | JP 2010-512208 A (CN SYSTEMS MEDIZINTECHNIK GMBH) 22 April 2010, entire text, all drawings & US 2008/0200785 A1 & CN 101621958 A | 1-9 |
| A | JP 2013-43026 A (UNIV. WASEDA) 04 March 2013, entire text, all drawings (Family: none) | 1-9 |
| A | JP 2015-116473 A (HYUNDAI MOTOR COMPANY) 25 June 2015, entire text, all drawings & US 2015/0164364 A1 & EP 2886051 A1 & CN 104720769 A | 1-9 |
| A | JP 2016-202516 A (KANSAI UNIVERSITY) 08 December 2016, entire text, all drawings (Family: none) | 1-9 |

☐ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 03 July 2018 (03.07.2018) | 10 July 2018 (10.07.2018) |

| Name and mailing address of the ISA/<br>Japan Patent Office<br>3-4-3, Kasumigaseki, Chiyoda-ku,<br>Tokyo 100-8915, Japan | Authorized officer<br><br><br>Telephone No. |
| --- | --- |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013153783 A
- JP 2016202516 A
- JP HEI5237119 A
- JP 2010532208 T
- JP 2005087543 A